# EUROPEAN PATENT APPLICATION

(11) **EP 4 528 747 A1**
(43) Date of publication of application: **26.03.2025**
(21) Application number: 24201613.7
(22) Date of filing: 20.09.2024
(51) Int. Cl.: G16H 40/00, G16H 15/00, G06F 16/903, G16C 20/90, G16H 10/40, G01N 35/00

(54) **METHODS AND SYSTEMS FOR LABORATORY SAMPLE TRACKING**

(30) Priority: 25.09.2023 US 202363629956 P
(71) Applicant: Thermo Electron Manufacturing Limited, Hemel Hempstead Hertfordshire HP2 7GE (GB)
(72) Inventor: HADFIELD, Max, Hemel Hempstead, HP2 7GE (GB)
(74) Representative: Boult Wade Tennant LLP

(57) **Abstract**

Disclosed herein are scientific instrument support systems, as well as related methods, computing devices, and computer-readable media. For example, in some embodiments, a method is provided that includes generating a sample identifier for a sample, receiving sample information for the sample, and generating a data structure ledger for the sample. The data structure ledger includes an initial ledger block with the sample identifier and the sample information. The method also includes receiving experiment data relating to the sample, generating an experiment ledger block in the data structure ledger. The method further includes receiving a request for a report corresponding to the sample, querying the data structure ledger to receive ledger data corresponding to the request, and generating the report based on the ledger data received in response to the query.

## Description

### Cross-Reference to Related Applications

This application claims priority to co-pending U.S. Provisional Patent Application No. 63/629,956, filed September 25, 2023, the entire contents of which is incorporated herein by reference.

### Background

Clinical and testing laboratories receive samples for analysis, which may be divided into aliquots. Each sample (each aliquot) may be prepared and/or analyzed by several scientific instruments including, for example, chromatographs (e.g., gas, liquid, etc.), spectrometers, and the like.

### Brief Description of the Drawings

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 is a block diagram of an example scientific instrument support system for performing support operations, in accordance with various embodiments.
FIG. 2 is a block diagram of an example scientific instrument support module for performing support operations, in accordance with various embodiments.
FIG. 3 is an example of a graphical user interface that may be used in the performance of some or all of the support methods disclosed herein, in accordance with various embodiments.
FIG. 4 is a block diagram of an example computing device that may perform some or all of the scientific instrument support methods disclosed herein, in accordance with various embodiments.
FIG. 5 is a block diagram of an example scientific instrument support system in which some or all of the scientific instrument support methods disclosed herein may be performed, in accordance with various embodiments.
FIG. 6 is a flow diagram of an example method of performing support operations, in accordance with various embodiments.
FIGS. 7A-7D are examples of graphical user interfaces that may be used in the performance of some or all of the support methods disclosed herein, in accordance with various embodiments.
FIG. 8 is an example of a graphical user interface that may be used in the performance of some or all of the support methods disclosed herein, in accordance with various embodiments.

### Detailed Description

In laboratory settings, samples are processed and/or analyzed by one or more scientific instruments (e.g., via one or more aliquots), such as, for example, to determine the presence or absence of compounds within the sample, an amount of a compound within the sample, or the like. Each scientific instrument processing and/or analyzing the sample may use different software applications to conduct the analysis and generate analysis results. Accordingly, to generate a report for a sample, a technician manually gathers information from each of the scientific instruments and manually compiles the gathered information. Ensuring that the report includes all applicable results associated with the sample is cumbersome and error prone, as the technician must track a sample across different aliquots and/or scientific instructions as well as across results that may be generated by different software applications, which may use different ways to identify a sample. A laboratory information management system (LIMS) may be used to track a sample through the various experiments conducted by one or more scientific instruments. However, the LIMS may still face several of the above problems. The entries in such a LIMS are made manually by the technician, and the LIMS does not integrate with the scientific instruments. The different ways in which data is generated and stored by the LIMS and the scientific instruments may make it difficult to map results to a particular sample being tracked via the LIMS. Additionally, LIMS only stores a subset of the data associated with the sample. The data stored by LIMS may not be intrinsically linked to the metadata of the sample. Thus, not only is report generation therefore very cumbersome and is prone to errors but often makes inefficient use of laboratory resources, including instrument usage, consumable usage, data processing usage, bandwidth usage, and the like.

Accordingly, to address these and other issues with existing laboratory environments and associated management technology, disclosed herein are scientific instrument support systems, as well as related methods, computing devices, and computer-readable media. For example, in some embodiments, a method is provided that includes generating, with an processing device, a sample identifier for a sample, receiving, with the processing device, sample information for the sample, and receiving, with the processing device from a plurality of scientific instruments, experiment data relating to the sample and generating, with the processing device, a data structure ledger for the sample including the experiment data linked to the sample identifier and the sample information. The method also includes receiving a request for a report corresponding to the sample, querying the data structure ledger to receive ledger data corresponding to the request, and generating the report based on the ledger data received in response to the query.

In some embodiments, a scientific instrument support system includes one or a set of processing devices configured to generate a sample identifier for a sample, receive sample information for the sample, and generate a data structure ledger for the sample. The data structure ledger includes an initial ledger block with the sample identifier and the sample information. The one or a set of processing devices is also configured to generate an experiment ledger block in the data structure ledger. The experiment ledger block includes experiment data received from a scientific instrument. The one or a set of processing devices is further configured to receive a request for a report corresponding to the sample, query the data structure ledger to receive ledger data corresponding to the request, and generate the report based on the ledger data received in response to the query.

In some embodiments, a scientific instrument support apparatus includes a first logic to generate a sample identifier for a sample, second logic to receive sample information for the sample, and third logic to generate a data structure ledger for the sample. The data structure ledger includes an initial ledger block with the sample identifier and the sample information. The scientific instrument support apparatus also includes fourth logic to generate timestamped experiment ledger blocks in the data structure ledger. The experiment ledger blocks including experiment data received from a plurality of scientific instruments. The scientific instrument support apparatus further includes fifth logic to receive a request for a report corresponding to the sample, sixth logic to query the data structure ledger to receive ledger data corresponding to the request, and seventh logic to generate the report based on the ledger data received in response to the query.

The scientific instrument support embodiments disclosed herein may achieve improved performance relative to conventional approaches. For example, the described sample tracking methods and systems significantly improve sample information storage and retrieval for report generation. Specifically, the described methods and systems allow for easy integration of data from various scientific instrument analysis performed on a sample such that the data may be later accessed for generating reports. The embodiments disclosed herein thus provide improvements to scientific instrument technology (e.g., improvements in the computer technology supporting such scientific instruments, among other improvements). An overall laboratory platform (e.g., Ardia^{™} platform) may create opportunities for ensuring results data is written to/correlated with any necessary sample information to create an audit trail for samples.

Various ones of the embodiments disclosed herein may improve upon conventional approaches to achieve the technical advantages of higher throughput and data validation by improved data structure relationships between the various experiments conducted on the samples. Such technical advantages are not achievable by routine and conventional approaches, and all users of systems including such embodiments may benefit from these advantages (e.g., by assisting the user in the performance of a technical task, such as report generation for samples, by means of a guided human-machine interaction process). As discussed further herein, various aspects of the embodiments disclosed herein may improve the functionality of a computer itself; for example, the speed at which the sample data can be parsed and later retrieved. The computational and user interface features disclosed herein do not only involve the collection and comparison of information but apply new analytical and technical techniques to change the operation of the scientific support apparatus. The present disclosure thus introduces functionality that neither a conventional computing device, nor a human, could perform.

In the following detailed description, reference is made to the accompanying drawings that form a part hereof wherein like numerals designate like parts throughout, and in which is shown, by way of illustration, embodiments that may be practiced. It is to be understood that other embodiments may be utilized, and structural or logical changes may be made, without departing from the scope of the present disclosure. Therefore, the following detailed description is not to be taken in a limiting sense.

Various operations may be described as multiple discrete actions or operations in turn, in a manner that is most helpful in understanding the subject matter disclosed herein. However, the order of description should not be construed as to imply that these operations are necessarily order dependent. In particular, these operations may not be performed in the order of presentation. Operations described may be performed in a different order from the described embodiment. Various additional operations may be performed, and/or described operations may be omitted in additional embodiments.

For the purposes of the present disclosure, the phrases "A and/or B" and "A or B" mean (A), (B), or (A and B). For the purposes of the present disclosure, the phrases "A, B, and/or C" and "A, B, or C" mean (A), (B), (C), (A and B), (A and C), (B and C), or (A, B, and C). Although some elements may be referred to in the singular (e.g., "a processing device"), any appropriate elements may be represented by multiple instances of that element, and vice versa. For example, a set of operations described as performed by a processing device may be implemented with different ones of the operations performed by different processing devices.

The description uses the phrases "an embodiment," "various embodiments," and "some embodiments," each of which may refer to one or more of the same or different embodiments. Furthermore, the terms "comprising," "including," "having," and the like, as used with respect to embodiments of the present disclosure, are synonymous. When used to describe a range of dimensions, the phrase "between X and Y" represents a range that includes X and Y. As used herein, an "apparatus" may refer to any individual device, collection of devices, part of a device, or collections of parts of devices. The drawings are not necessarily to scale.

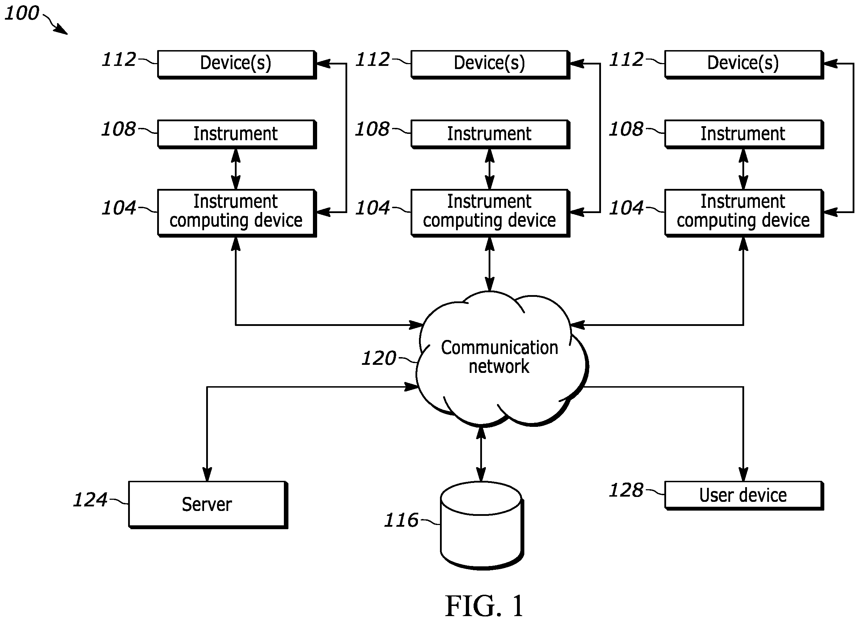

FIG. 1 is a block diagram of a scientific instrument support system 100 (henceforth referred to simply as support system 100) for performing support operations, in accordance with various embodiments. As illustrated in FIG. 1, the support system 100 includes a plurality of instrument computing devices 104 connected over a communication network 120. Each instrument computing device 104 is communicatively connected to a scientific instrument 108 and corresponding support devices 112. The support devices 112 are physical entities that perform some function of sample preparation and/or sample analysis. The support devices 112 may include, for example, sensors, detectors, actuators, spectrometers, spectrograms, oscilloscopes, electrometers, interferometers, and the like. The scientific instruments 108 are logical containers that include a collection of support devices 112. For example, support devices 112 work together to perform operations and produce results. The scientific instrument 108, including the collection of support devices 112, prepares or analyzes inputs, such as blood samples or other biological samples, semiconductors, chemical compounds, solutions, food and drug samples, and the like. The scientific instruments 108 may include, for example, liquid chromatography instruments, gas chromatography instruments, ion chromatography instruments, mass spectrometry instruments, trace elemental instruments (e.g., inductively coupled plasma mass spectrometry, inductively coupled plasma optical emission spectroscopy, atomic absorption, etc.), capillary electrophoresis instruments, spectroscopy instruments, and the like.

The support system 100 includes a database 116, a server 124, and a user device 128 communicatively coupled with the plurality of instrument computing devices 104 (and with each other) through the communication network 120. It should be understood that the support system 100 may include fewer or additional components than those illustrated in FIG. 1. For example, the support system 100 may include fewer or more instrument computing devices 104, multiple servers 124, multiple databases 116, or a combination thereof. In some instances, the database 116 may be implemented by the server 124 or a cloud service provider. The components of the support system 100 may also communicate through one or more intermediary devices not illustrated in FIG. 1.

The server 124 may serve as a "control hub" for the plurality of instrument computing devices 104. For example, the server 124 may track the status of each instrument computing device 104 to track the availability of and experiments being conducted on the scientific instruments 108. The server 124 may communicate with each of the instrument computing devices 104 by sending commands to the instrument computing devices 104 to write experiment data to data structure ledgers as further described below or may be responsible for writing experiment data output by a particular instrument to the data structure ledgers as further described below. For example, the server 124 may implement a server-based platform (e.g., the Ardia^{™} Platform from Thermo Fisher Scientific), which may be implemented as a cloud-based platform or using a private server. The server 124 may detect and register instrument computing devices 104 and may collect and regulate information output by the instrument computing devices 104. The server-based platform helps ensure sample information and experiment data is stored and accessible by laboratory equipment and devices. The server 124 may act as the control hub or facilitating hub (e.g., for centralized ledger examples) for the overall platform to facilitate an audit trail for samples and to ensure result data from experiments is written to and correlated with any necessary sample information (e.g., sample identifier), project information (e.g., project identifier), user information (e.g., user identifier), instrument information (e.g., instrument identifier), or the like to create an audit trail for samples.

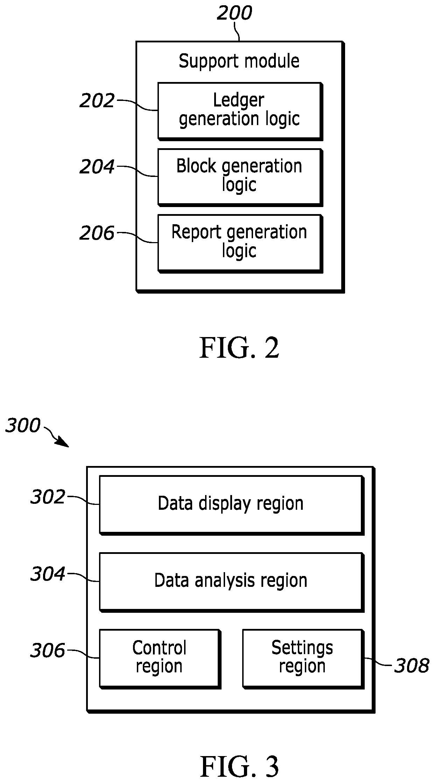

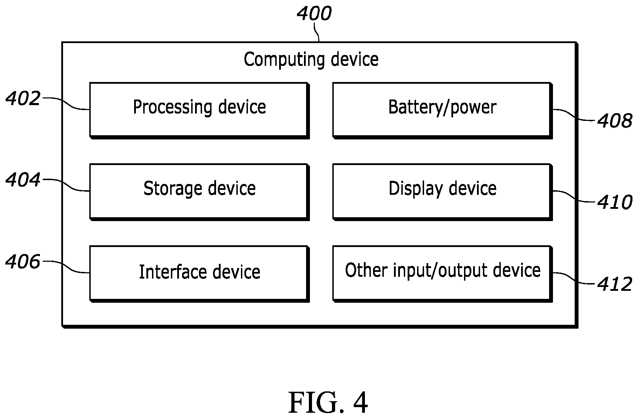

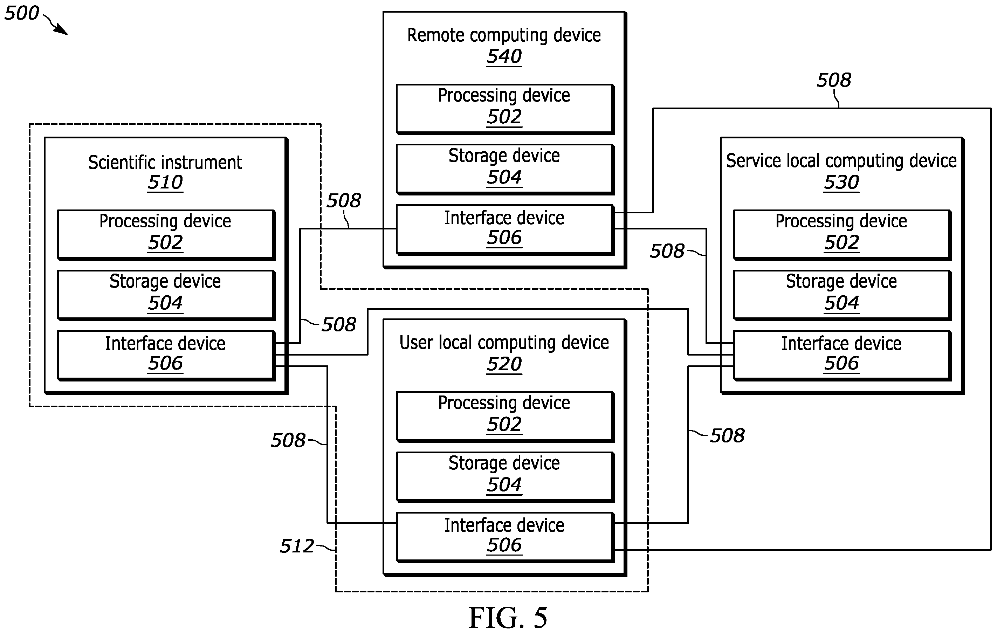

FIG. 2 is a block diagram of a scientific instrument support module 200 for performing support operations, in accordance with various embodiments. The scientific instrument support module 200 may be, for example, the server 124 and/or the instrument computing device 104. The scientific instrument support module 200 may be implemented by circuitry (e.g., including electrical and/or optical components), such as a programmed computing device. The logic of the scientific instrument support module 200 may be included in a single computing device or may be distributed across multiple computing devices that are in communication with each other as appropriate. Examples of computing devices that may, singly or in combination, implement the scientific instrument support module 200 are discussed herein with reference to the computing device 400 of FIG. 4, and examples of systems of interconnected computing devices, in which the scientific instrument support module 200 may be implemented across one or more of the computing devices, is discussed herein with reference to the scientific instrument support system 500 of FIG. 5.

The scientific instrument support module 200 may include a ledger logic 202, a block logic 204, and a report logic 206. As used herein, the term "logic" may include an apparatus that is to perform a set of operations associated with the logic. For example, any of the logic elements included in the support module 200 may be implemented by one or more computing devices programmed with instructions to cause one or more processing devices of the computing devices to perform the associated set of operations. In a particular embodiment, a logic element may include one or more non-transitory computer-readable media having instructions thereon that, when executed by one or more processing devices of one or more computing devices, cause the one or more computing devices to perform the associated set of operations. As used herein, the term "module" may refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module.

The ledger logic 202 may create a data structure ledger for each sample received by a laboratory or for each project (including multiple samples) conducted by the laboratory. In one example, the data structure ledger may be a blockchain ledger, for example, a distributed blockchain ledger (peer-to-peer, federated, public, public, permissioned, permissionless, etc.) or a centralized blockchain ledger. In other examples, the data structure ledger may be distributed or centralized and may be based on various ledger technologies and infrastructures, not limited to blockchain, such as, for example, IOTA, cache graphs, directed acyclic graphs (DAG), distributed hash tables, Merkle trees, an Adelson-Velsky and Landis (AVL) tree, a B-tree, stacks, queues, a hybrid combination of different data structures and infrastructures, or the like. In some examples, the data structure ledger may be a relational table, a hash table, a linked list, and/or the like. The ledger logic 202 may automatically generate a unique sample identifier for each sample being tracked. The ledger logic 202 receives sample information relating to the sample and correlates the sample information with the sample identifier or other identifier, for example, using a data structure object.

The block logic 204 may generate an experiment block in the data structure ledger including experiment data relating to the sample. The block logic 204 may receive experiment data corresponding to an experiment performed on the sample by a scientific instrument 108. The block logic 204 generates an experiment data block that includes a reference to the data structure ledger or a node or block within the data structure ledger. For example, the experiment ledger block may include a hash with a reference to a previous block in the data structure ledger for the block. An experiment block is, for example, an entry in the data structure ledger and need not be in the form of a block of a block chain. The experiment block may include for example, a table entry, a pointer data structure entry, and/or the like. The experiment block may be created using a block object that may be similar to the data structure object discussed above.

The report logic 206 may generate a report from the data structure logic based on a query from a user. The query may include a request for data associated with one or more experiments performed on a sample. The report logic 206 retrieves the relevant data from the data structure ledger and automatically generates a report based on the data.

The scientific instrument support methods disclosed herein may include interactions with a human user (e.g., via the user local computing device 520 discussed herein with reference to FIG. 5). These interactions may include providing information to the user (e.g., information regarding the operation of a scientific instrument such as the scientific instrument 510 of FIG. 5, information regarding a sample being analyzed or other test or measurement performed by a scientific instrument, information retrieved from a local or remote database, or other information) or providing an option for a user to input commands (e.g., to control the operation of a scientific instrument such as the scientific instrument 510 of FIG. 5, or to control the analysis of data generated by a scientific instrument), queries (e.g., to a local or remote database), or other information. In some embodiments, these interactions may be performed through a graphical user interface (GLTI) that includes a visual display on a display device (e.g., the display device 410 discussed herein with reference to FIG. 4) that provides outputs to the user and/or prompts the user to provide inputs (e.g., via one or more input devices, such as a keyboard, mouse, trackpad, or touchscreen, included in the other I/O devices 412 discussed herein with reference to FIG. 4). The scientific instrument support systems disclosed herein may include any suitable GUIs for interaction with a user.

FIG. 3 depicts an example GUI 300 that may be used in the performance of some or all of the support methods disclosed herein, in accordance with various embodiments. As noted above, the GUI 300 may be provided on a display device (e.g., the display device 410 discussed herein with reference to FIG. 4) of a computing device (e.g., the computing device 400 discussed herein with reference to FIG. 4) of a scientific instrument support system (e.g., the scientific instrument support system 500 discussed herein with reference to FIG. 5), and a user may interact with the GUI 300 using any suitable input device (e.g., any of the input devices included in the other I/O devices 412 discussed herein with reference to FIG. 4) and input technique (e.g., movement of a cursor, motion capture, facial recognition, gesture detection, voice recognition, actuation of buttons, etc.).

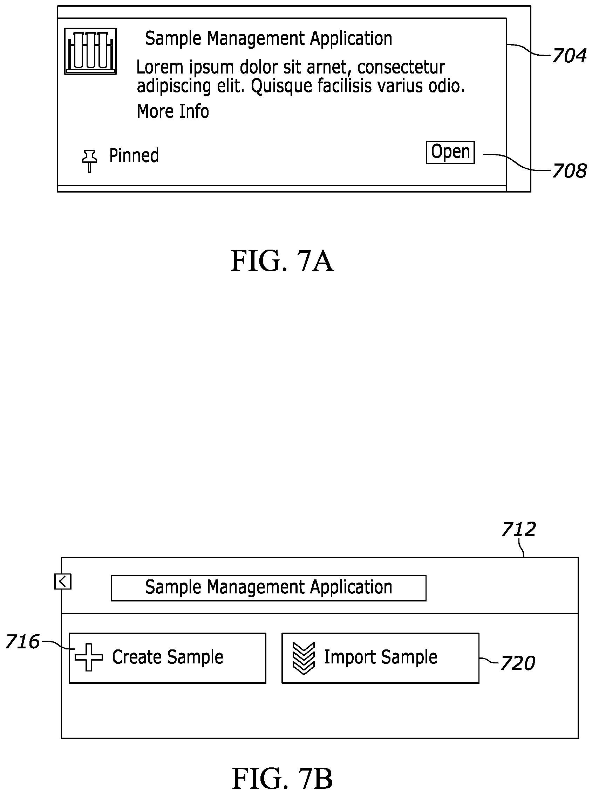

The GUI 300 may include a data display region 302, a data analysis region 304, a scientific instrument control region 306, and a settings region 308. The particular number and arrangement of regions depicted in FIG. 3 is simply illustrative, and any number and arrangement of regions, including any desired features, may be included in a GUI 300. Representations like those of FIGS. 7A-8 may be included in any appropriate region of the GUI 300 (e.g., in the data display region 302 or the data analysis region 304 of the GUI 300).

The data display region 302 may display data generated by a scientific instrument (e.g., the scientific instrument 510 discussed herein with reference to FIG. 5). For example, the data display region 302 may display experiment data, for example, the results of an experiment, the sample identifier of the sample, the experiment parameters, or the like.

The data analysis region 304 may display the results of data analysis (e.g., the results of analyzing the data illustrated in the data display region 302 and/or other data). For example, the data analysis region 304 may display a report generated based on the data structure ledger of the sample. In some embodiments, the data display region 302 and the data analysis region 304 may be combined in the GUI 300 (e.g., to include data output from a scientific instrument, and some analysis of the data, in a common graph or region).

The scientific instrument control region 306 may include options that allow the user to control a scientific instrument (e.g., the scientific instrument 510 discussed herein with reference to FIG. 5). For example, the scientific instrument control region 306 may include control features of a chromatography instrument, a spectrometry instrument, or the like.

The settings region 308 may include options that allow the user to control the features and functions of the GUI 300 (and/or other GUIs) and/or perform common computing operations with respect to the data display region 302 and data analysis region 304 (e.g., saving data on a storage device, such as the storage device 404 discussed herein with reference to FIG. 4, sending data to another user, labeling data, etc.). For example, the settings region 308 may include options on how to display the sample information and/or a query input feature.

As noted above, the scientific instrument support module 200 may be implemented by one or more computing devices. FIG. 4 is a block diagram of a computing device 400 that may perform some or all of the scientific instrument support methods disclosed herein, in accordance with various embodiments. In some embodiments, the scientific instrument support module 200 may be implemented by a single computing device 400 or by multiple computing devices 400. Further, as discussed below, a computing device 400 (or multiple computing devices 400) that implements the scientific instrument support module 200 may be part of one or more of the scientific instrument 510, the user local computing device 520, the service local computing device 530, or the remote computing device 540 of FIG. 5.

The computing device 400 of FIG. 4 is illustrated as having a number of components, but any one or more of these components may be omitted or duplicated, as suitable for the application and setting. In some embodiments, some or all of the components included in the computing device 400 may be attached to one or more motherboards and enclosed in a housing (e.g., including plastic, metal, and/or other materials). In some embodiments, some these components may be fabricated onto a single system-on-a-chip (SoC) (e.g., an SoC may include one or more processing devices 402 and one or more storage devices 404). Additionally, in various embodiments, the computing device 400 may not include one or more of the components illustrated in FIG. 4, but may include interface circuitry (not shown) for coupling to the one or more components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface) . For example, the computing device 400 may not include a display device 410, but may include display device interface circuitry (e.g., a connector and driver circuitry) to which a display device 410 may be coupled.

The computing device 400 may include a processing device 402 (e.g., one or more processing devices). As used herein, the term "processing device" may refer to any device or portion of a device that processes electronic data from registers and/or memory to transform that electronic data into other electronic data that may be stored in registers and/or memory. The processing device 402 (i.e., electronic processing device or electronic processor) may include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 400 may include a storage device 404 (e.g., one or more storage devices). The storage device 404 may include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some embodiments, the storage device 404 may include memory that shares a die with a processing device 402. In such an embodiment, the memory may be used as cache memory and may include embedded dynamic random access memory (eDRAM) or spin transfer torque magnetic random access memory (STT-MRAM), for example. In some embodiments, the storage device 404 may include non-transitory computer readable media having instructions thereon that, when executed by one or more processing devices (e.g., the processing device 402), cause the computing device 400 to perform any appropriate ones of or portions of the methods disclosed herein.

The computing device 400 may include an interface device 406 (e.g., one or more interface devices 406). The interface device 406 may include one or more communication chips, connectors, and/or other hardware and software to govern communications between the computing device 400 and other computing devices. For example, the interface device 406 may include circuitry for managing wireless communications for the transfer of data to and from the computing device 400. The term "wireless" and its derivatives may be used to describe circuits, devices, systems, methods, techniques, communications channels, etc., that may communicate data through the use of modulated electromagnetic radiation through a nonsolid medium. The term does not imply that the associated devices do not contain any wires, although in some embodiments they might not. Circuitry included in the interface device 406 for managing wireless communications may implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In some embodiments, circuitry included in the interface device 4006 for managing wireless communications may operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In some embodiments, circuitry included in the interface device 4006 for managing wireless communications may operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In some embodiments, circuitry included in the interface device 406 for managing wireless communications may operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In some embodiments, the interface device 406 may include one or more antennas (e.g., one or more antenna arrays) to receipt and/or transmission of wireless communications.

In some embodiments, the interface device 406 may include circuitry for managing wired communications, such as electrical, optical, or any other suitable communication protocols. For example, the interface device 406 may include circuitry to support communications in accordance with Ethernet technologies. In some embodiments, the interface device 406 may support both wireless and wired communication, and/or may support multiple wired communication protocols and/or multiple wireless communication protocols. For example, a first set of circuitry of the interface device 406 may be dedicated to shorter-range wireless communications such as Wi-Fi or Bluetooth, and a second set of circuitry of the interface device 406 may be dedicated to longer-range wireless communications such as global positioning system (GPS), EDGE, GPRS, CDMA, WiMAX, LTE, EV-DO, or others. In some embodiments, a first set of circuitry of the interface device 406 may be dedicated to wireless communications, and a second set of circuitry of the interface device 406 may be dedicated to wired communications.

The computing device 400 may include battery/power circuitry 408. The battery/power circuitry 408 may include one or more energy storage devices (e.g., batteries or capacitors) and/or circuitry for coupling components of the computing device 400 to an energy source separate from the computing device 400 (e.g., AC line power).

The computing device 400 may include a display device 410 (e.g., multiple display devices). The display device 410 may include any visual indicators, such as a heads-up display, a computer monitor, a projector, a touchscreen display, a liquid crystal display (LCD), a light-emitting diode display, or a flat panel display.

The computing device 400 may include other input/output (I/O) devices 412. The other I/O devices 412 may include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 400, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 400 may have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

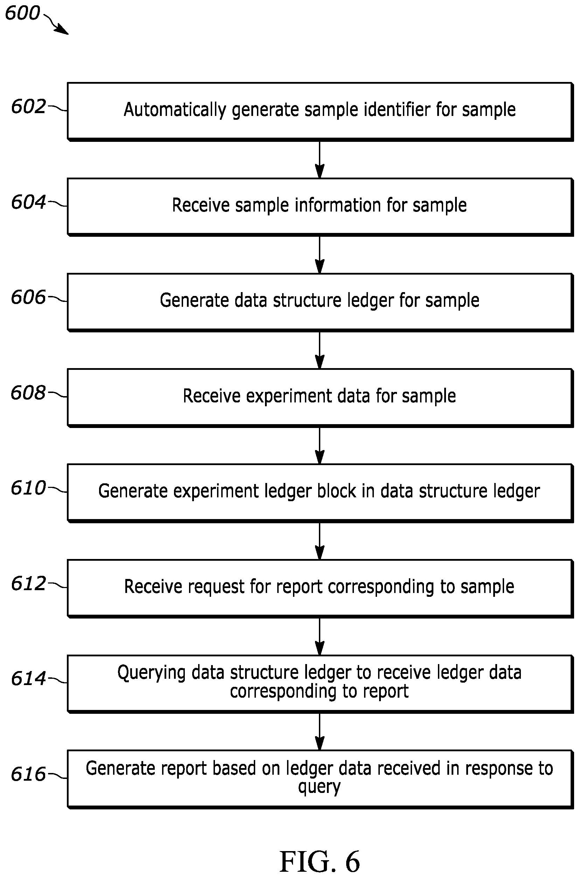

One or more computing devices implementing any of the scientific instrument support modules or methods disclosed herein may be part of a scientific instrument support system. FIG. 5 is a block diagram of an example scientific instrument support system 500 in which some or all of the scientific instrument support methods disclosed herein may be performed, in accordance with various embodiments. The scientific instrument support modules and methods disclosed herein (e.g., the scientific instrument support system 100 of FIG. 1, the scientific instrument support module 200 of FIG. 2 and the method 600 of FIG. 6) may be implemented by one or more of the scientific instrument 510, the user local computing device 520, the service local computing device 530, or the remote computing device 540 of the scientific instrument support system 500.

Any of the scientific instrument 510, the user local computing device 520, the service local computing device 530, or the remote computing device 540 may include any of the embodiments of the computing device 400 discussed herein with reference to FIG. 4, and any of the scientific instrument 510, the user local computing device 520, the service local computing device 530, or the remote computing device 540 may take the form of any appropriate ones of the embodiments of the computing device 400 discussed herein with reference to FIG. 4.

The scientific instrument 510, the user local computing device 520, the service local computing device 530, or the remote computing device 540 may each include a processing device 502, a storage device 504, and an interface device 506. The processing device 502 may take any suitable form, including the form of any of the processing devices 402 discussed herein with reference to FIG. 4, and the processing devices 502 included in different ones of the scientific instrument 510, the user local computing device 520, the service local computing device 530, or the remote computing device 540 may take the same form or different forms. The storage device 504 may take any suitable form, including the form of any of the storage devices 504 discussed herein with reference to FIG. 4, and the storage devices 504 included in different ones of the scientific instrument 510, the user local computing device 520, the service local computing device 530, or the remote computing device 540 may take the same form or different forms. The interface device 506 may take any suitable form, including the form of any of the interface devices 406 discussed herein with reference to FIG. 4, and the interface devices 506 included in different ones of the scientific instrument 510, the user local computing device 520, the service local computing device 530, or the remote computing device 540 may take the same form or different forms.

The scientific instrument 510, the user local computing device 520, the service local computing device 530, and the remote computing device 540 may be in communication with other elements of the scientific instrument support system 500 via communication pathways 508. The communication pathways 508 may communicatively couple the interface devices 506 of different ones of the elements of the scientific instrument support system 500, as shown, and may be wired or wireless communication pathways (e.g., in accordance with any of the communication techniques discussed herein with reference to the interface devices 406 of the computing device 400 of FIG. 4). The particular scientific instrument support system 500 depicted in FIG. 5 includes communication pathways between each pair of the scientific instrument 510, the user local computing device 520, the service local computing device 530, and the remote computing device 540, but this "fully connected" implementation is simply illustrative, and in various embodiments, various ones of the communication pathways 508 may be absent. For example, in some embodiments, a service local computing device 530 may not have a direct communication pathway 508 between its interface device 506 and the interface device 506 of the scientific instrument 510, but may instead communicate with the scientific instrument 510 via the communication pathway 508 between the service local computing device 530 and the user local computing device 520 and the communication pathway 508 between the user local computing device 520 and the scientific instrument 510.

The scientific instrument 510 may include any appropriate scientific instrument, such as a liquid chromatography instruments, gas chromatography instruments, ion chromatography instruments, mass spectrometry instruments, trace elemental instruments (e.g., inductively coupled plasma mass spectrometry, inductively coupled plasma optical emission spectroscopy, atomic absorption, etc.), capillary electrophoresis instruments, spectroscopy instruments, and the like.

The user local computing device 520 may be a computing device (e.g., in accordance with any of the embodiments of the computing device 400 discussed herein) that is local to a user of the scientific instrument 510. In some embodiments, the user local computing device 520 may also be local to the scientific instrument 510, but this need not be the case; for example, a user local computing device 520 that is in a user's home or office may be remote from, but in communication with, the scientific instrument 510 so that the user may use the user local computing device 520 to control and/or access data from the scientific instrument 510. In some embodiments, the user local computing device 520 may be a laptop, smartphone, or tablet device. In some embodiments the user local computing device 520 may be a portable computing device. In some embodiments, the user local computing device 520 may be used for sample creation including receiving sample information for a sample to be analyzed by the laboratory.

The service local computing device 530 may be a computing device (e.g., in accordance with any of the embodiments of the computing device 400 discussed herein) that is local to an entity that services the scientific instrument 510. For example, the service local computing device 530 may be local to a manufacturer of the scientific instrument 510 or to a third-party service company. In some embodiments, the service local computing device 530 may communicate with the scientific instrument 510, the user local computing device 520, and/or the remote computing device 540 (e.g., via a direct communication pathway 508 or via multiple "indirect" communication pathways 508, as discussed above) to receive data regarding the operation of the scientific instrument 510, the user local computing device 520, and/or the remote computing device 540 (e.g., the results of self-tests of the scientific instrument 510, calibration coefficients used by the scientific instrument 510, the measurements of sensors associated with the scientific instrument 510, etc.). In some embodiments, the service local computing device 530 may communicate with the scientific instrument 510, the user local computing device 520, and/or the remote computing device 540 (e.g., via a direct communication pathway 508 or via multiple "indirect" communication pathways 508, as discussed above) to transmit data to the scientific instrument 510, the user local computing device 520, and/or the remote computing device 540 (e.g., to update programmed instructions, such as firmware, in the scientific instrument 510, to initiate the performance of test or calibration sequences in the scientific instrument 510, to update programmed instructions, such as software, in the user local computing device 520 or the remote computing device 540, etc.). A user of the scientific instrument 510 may utilize the scientific instrument 510 or the user local computing device 520 to communicate with the service local computing device 530 to report a problem with the scientific instrument 510 or the user local computing device 520, to request a visit from a technician to improve the operation of the scientific instrument 510, to order consumables or replacement parts associated with the scientific instrument 510, or for other purposes. The service local computing device 530 may generate the data structure ledger and generate reports based on the data retrieved from the data structure ledger.

The remote computing device 540 may be a computing device (e.g., in accordance with any of the embodiments of the computing device 400 discussed herein) that is remote from the scientific instrument 510 and/or from the user local computing device 520. In some embodiments, the remote computing device 540 may be included in a datacenter or other large-scale server environment. In some embodiments, the remote computing device 540 may include network-attached storage (e.g., as part of the storage device 504). The remote computing device 5040 may store data generated by the scientific instrument 510, perform analyses of the data generated by the scientific instrument 510 (e.g., in accordance with programmed instructions), facilitate communication between the user local computing device 520 and the scientific instrument 510, and/or facilitate communication between the service local computing device 530 and the scientific instrument 510. The remote computing device 540 may be used to query the data structure ledger and to generate reports based on the data retrieved from the data structure ledger.

In some embodiments, one or more of the elements of the scientific instrument support system 500 illustrated in FIG. 5 may not be present. Further, in some embodiments, multiple ones of various ones of the elements of the scientific instrument support system 500 of FIG. 5 may be present. For example, a scientific instrument support system 500 may include multiple user local computing devices 520 (e.g., different user local computing devices 520 associated with different users or in different locations). In another example, a scientific instrument support system 500 may include multiple scientific instruments 510, all in communication with service local computing device 530 and/or a remote computing device 540; in such an embodiment, the service local computing device 530 may monitor these multiple scientific instruments 510, and the service local computing device 530 may cause updates or other information may be "broadcast" to multiple scientific instruments 510 at the same time. Different ones of the scientific instruments 510 in a scientific instrument support system 500 may be located close to one another (e.g., in the same room) or farther from one another (e.g., on different floors of a building, in different buildings, in different cities, etc.). In some embodiments, a scientific instrument 510 may be connected to an Internet-of-Things (IoT) stack that allows for command and control of the scientific instrument 510 through a web-based application, a virtual or augmented reality application, a mobile application, and/or a desktop application. Any of these applications may be accessed by a user operating the user local computing device 520 in communication with the scientific instrument 510 by the intervening remote computing device 540. In some embodiments, a scientific instrument 510 may be sold by the manufacturer along with one or more associated user local computing devices 520 as part of a local scientific instrument computing unit 512.

In some embodiments, different ones of the scientific instruments 510 included in a scientific instrument support system 500 may be different types of scientific instruments 510; for example, one scientific instrument 510 may be a chromatography instrument, while another scientific instrument 510 may be a spectrometry instrument. In some such embodiments, the remote computing device 540 and/or the user local computing device 520 may combine data from different types of scientific instruments 510 included in a scientific instrument support system 500.

FIG. 6 is a flow diagram of a method 600 of performing support operations, in accordance with various embodiments. Although the operations of the method 600 may be illustrated with reference to particular embodiments disclosed herein (e.g., the scientific instrument support modules 200 discussed herein with reference to FIG. 2, the GUI 300 discussed herein with reference to FIG. 3, the computing devices 400 discussed herein with reference to FIG. 4, and/or the scientific instrument support system 500 discussed herein with reference to FIG. 5), the method 600 may be used in any suitable setting to perform any suitable support operations. Operations are illustrated once each and in a particular order in FIG. 6, but the operations may be reordered and/or repeated as desired and appropriate (e.g., different operations performed may be performed in parallel, as suitable).

At 602, the method 600 includes automatically generating a sample identifier for a sample. A sample is a substance received at a laboratory for chemical and/or biological analysis. For example, a soil sample may be received for analysis to detect the presence or amount of a nutrient or chemical, a wine sample may be received for analysis to detect the presence or amount of a chemical, or the like. The ledger logic 202 may generate one or more graphical user interfaces (GLTIs) to facilitate creation of a sample record and for collecting information relating to the sample (e.g., when the sample was collected, where the sample was collected from, how the sample was collected, etc.). Examples of sample creation GUIs generated by the ledger logic 202 are illustrated in FIGS. 7A-7D. As shown in FIG. 7A illustrates an application screen GUI 704 that allows a user to select a sample management application. The application screen GUI 704 includes an open icon 708 that is configured to be selected by, for example, clicking on the open icon 708.

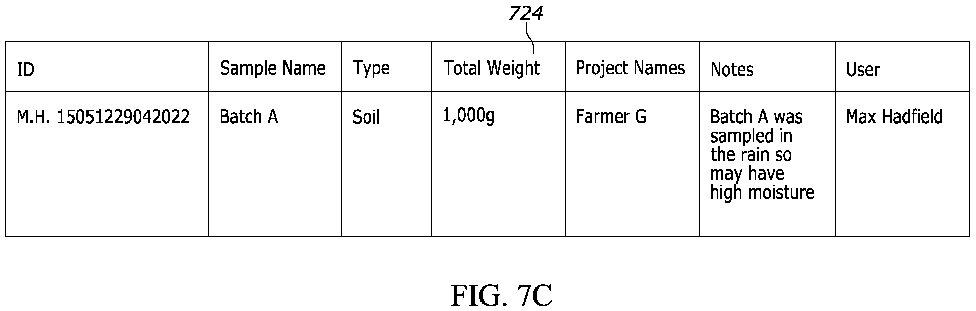

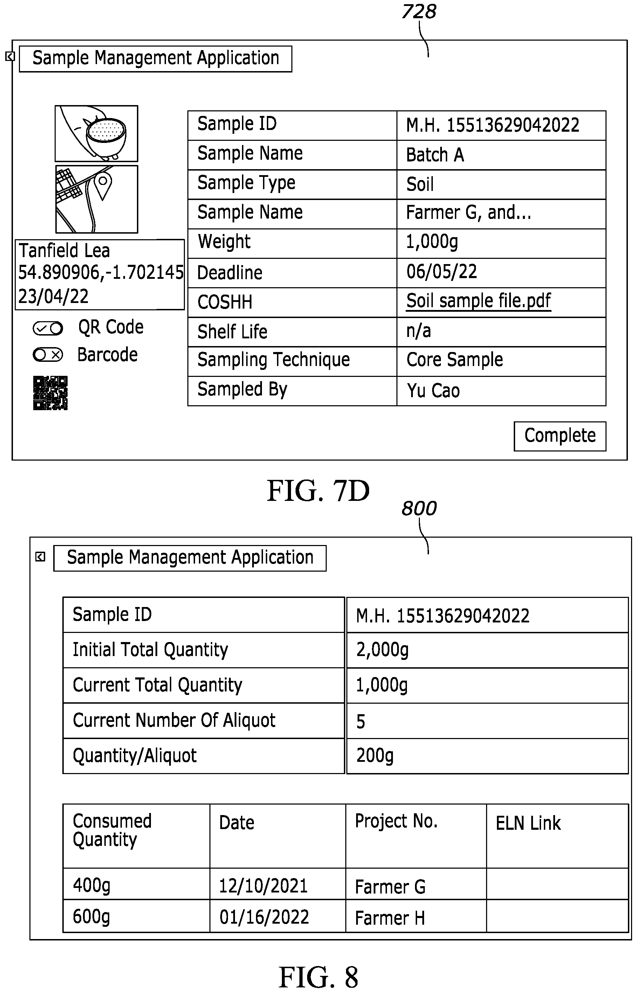

In response to receiving a selection of the open icon 708, the ledger logic 202 generates a sample creation GUI 712 (shown in FIG. 7B) that facilitates sample creation. Referring to FIG. 7B, the sample creation GUI 712 includes a create sample icon 716 and an import sample icon 720. Similar to the open icon 708, the create sample icon 716 and the import sample icon 720 are configured to be selected by, for example, clicking on the create sample icon 716 and the import sample icon 720, respectively. In response to receiving a selection of the create sample icon 716, the ledger logic 202 generates a sample information GUI 724 (shown in FIG. 7C). In response to receiving a selection of the import sample icon 720, the ledger logic 202 imports a previously created sample, for example, from a different application or from a previously created sample. Referring to FIG. 7C, the sample information GUI 724 includes one or more fields for receiving sample information. The sample information received via the GUI 724 may include, for example, a sample name, a sample type, a total weight of the sample, a project name for the sample, notes for the sample, a user handling the sample, and/or the like. FIG. 7D illustrates another sample information GUI 728 that may be generated in response to receiving a selection of the create sample icon 716. The sample information GUI 728 includes one or more fields for receiving sample information. The sample information received via the GUI 728 may include information that can be received on the sample information GUI 724 and also, for example, a deadline for the sample, a safety label of the sample (e.g., controlled substance hazardous to health warning), a shelf life of the sample, a sampling technique of the sample, a sampling entity for the sample, and/or the like.

As shown in FIG. 7C and 7D, the ledger logic 202 automatically generates the sample identifier, for example, in response to receiving a selection of the create sample icon 716. In other examples, the ledger logic 202 generates the sample identifier in response to other triggers, such as, for example, other inputs from the user. The sample identifier may be a numeric or alphanumeric identifier. In some examples, a barcode, QR code, or the like in addition to the numeric or alphanumeric identifier and/or encoding the numeric and alphanumeric identifier is also generated. In some examples, the sample identifier is randomly or semi-randomly generated hash value and is unique for the sample. In some examples, the generated barcode, QR code, or the like may be printed on labels, for example, using a label printer, and applied to sample containers containing each sample (or aliquot). Scanning devices (e.g., barcode/QR code scanners) may be used to quickly identify a sample by scanning the barcode, QR code, or the like attached to the sample container.

At 604, the method 600 includes receiving sample information for the sample. As shown in FIGS. 7C and 7D, the ledger logic 202 may receive sample information via one of the GUIs 724, 728.

At 606, the method 600 includes generating a data structure ledger for the sample. Generating the data structure leger for the sample may include generating the data structure ledger for a project including the sample. The project may include additional samples. The data structure ledger includes an initial ledger block (e.g., initial ledger entry) with the sample identifier and the sample information. The ledger logic 202 generates the data structure ledger with the initial ledger block as, for example, a genesis block of the data structure ledger. In one example, the data structure ledger is a centralized ledger such that the data structure ledger for the sample may be stored in the database 116. In other example, the data structure ledger is a distributed ledger such that each instrument computing device 104, the database 116, the server 124, and/or the user device 128 may each store a copy of the data structure ledger. In one example, the sample identifier may be used as the index to retrieve the data structure ledger from the database 116. In other example, a different index, for example, a hash of the initial block, a hash of the sample identifier, or the like may be used as the index to retrieve the data structure ledger from the database 116. In one example, the genesis block is generated by the ledger logic 202 using a data structure object. The data structure object may include a sample identifier field and a sample information field such that the sample identifier is correlated with the sample information in the genesis block. The genesis block is an instance of the data structure object for a particular sample.

At 608, the method 600 includes receiving, from a scientific instrument 510, experiment data relating to the sample, such as, for example, in real-time performance of an analytical workflow on the sample by the scientific instrument and/or in response to completion of an analytical workflow on the sample by the scientific instrument. The experiment data may include the sample identifier. The analytical workflow performed on the sample by the scientific instrument may include, for example, preparation of the sample for an experiment (e.g., dissolving the sample in a solution, injection of the solution into another scientific instrument, and/or the like), completion of an analysis or experiment on the sample, or the like. The experiment data may be received by the block logic 204. The experiment data includes information relating to the analytical workflow, for example, the analytical method carried out, the instrument used, the identity of the user performing the experiment (for example, one or more user identifiers), the location of the experiment, the time of the experiment, one or more project identifiers, a result of an experiment, an identifier of a scientific instrument, an identifier of an experiment performed on the sample, and/or the like.

At 610, the method 600 includes generating an experiment ledger block in the data structure ledger. The experiment ledger block includes at least some of the received experiment data and/or data generated based on the same. The block logic 204 generates the experiment ledger block using, for example, a block object. The block object may be similar to the data structure object described above and may include a sample identifier field and an experiment data field such that the sample identifier and other identifiers (e.g., project, user, instrument, or the like) are correlated with the experiment data in the experiment ledger block. The experiment ledger block is an instance of the block object. In some examples, the experiment ledger block may be similar to the initial ledger block with the sample information replaced with the experiment data. In some examples, a newly created experiment ledger block may include a pointer to the initial ledger block if no experiment ledger block was previously created or to a last-in experiment ledger block if one or more experiment ledger blocks were previously created. The experiment ledger blocks are therefore added to an end of blockchain, a linked list, or the like. In some examples, a newly created experiment ledger block may include a pointer to the initial ledger block regardless of whether a previous experiment ledger block is created. In other examples, a different pointer structure may be used in the experiment ledger block to create different forms of data structures. In some examples, the method 600 receives experiment data relating to the sample from a plurality of scientific instruments. The generated data structure ledger for the sample includes the experiment data linked to the sample identifier and the sample information. In at least one instance, the data structure ledger comprises a blockchain structure.

In some examples, a separate data structure ledger is created for each sample as described above. In other examples, all sample data is stored in a single data structure ledger. In these examples, the genesis block may be unique to a laboratory (or an organization running several laboratories) or a project. Information for each sample including the sample information and the experiment data may be included in data structure blocks or entries of the data structure ledger for the laboratory. As discussed above, the data structure ledger may be a centralized ledger, for example, stored in the database 116. In this example, the centralized ledger is accessed from the database 116 by each requesting computing entity (i.e., the server 124, the user device 128, the instrument computing devices 104, etc.) and edited or queried accordingly. In other examples, the data structure ledger may be a decentralized ledger such that a copy of the data structure ledger for each sample is stored in each (or one or more) of the instrument computing devices 104, the database 116, the server 124, and the user device 128. Each computing entity edits a locally stored copy of the data structure ledger to add a corresponding block (or entry) for a sample. All the copies of the data structure ledger may be reconciled at regular intervals or on a transaction basis. Each block of the data structure ledger may be encrypted using, for example, cryptographic hash functions and digital signatures. Additionally, each block of the data structure ledger is immutable such that once created, the block may not be altered. A decentralized ledger provided additional advantages of being secure and providing additional audit and control capabilities compared to other types of data structures as the ledger block are immutable. In some examples, the data is only changeable by a 'privileged user.' A privileged user may be a laboratory administrator or other user that has special privileges to correct stored records in the laboratory. The correction performed by the privileged user may also be recorded to provide a secure audit trail. The data structure ledger can be exported to other systems, for example, LIMS, an external data management system, or the like.

At 612, the method 600 includes receiving a request for a report corresponding to the sample. The request for the report may be received by the report logic 206 via a graphical user interface for querying the database 116. The request may be in the form of, for example, a structured query language (SQL) query, a NoSQL query, or the like. In some examples, the request for the report may be received from a smartphone application running on a mobile telephone (for example, the user device 128). In some embodiments, a report may be requested based on other criteria. For example, the a request (e.g., a second request) may be received corresponding to a scientific instrument, an experiment, a user, a laboratory, a group of instruments, a group of users, or the like.

At 614, the method 600 includes querying the data structure ledger to receive ledger data corresponding to the request. The report logic 206 queries the database 116 based on the request for the report to retrieve ledger data corresponding to the request. The ledger data includes the relevant data from the data structure ledger for generating the requested report. The query may specify one or more sample identifiers to retrieve ledger data corresponding to the one or more sample identifiers. In some examples, the query may be directed to a plurality of data structure ledgers associated with a plurality of samples. The report logic 206 may parse data from the plurality of data structure ledgers with data corresponding to the queried criteria (e.g., a scientific instrument, an experiment, or the like). For example, the data may be parsed based on a plurality of the blocks including the identifier of the criteria (e.g., an identifier of the scientific instrument, an identifier of an experiment, or the like).

At 616, the method 600 includes generating the report based on the ledger data received in response to the query. The report logic 206 generates the report, for example, a pre-defined template. The report may be generated based on the data for the project including multiple samples. The report may also be generated for each sample separately or for each experiment separately. Since the experiment data is correlated to the identifiers, reports may be easily generated by using any of the identifiers (e.g., sample identifier, project identifier, user identifier, instrument identifier, or the like). FIG. 8 illustrates one example of a report 800 generated by the report logic 206. The report 800 is related to quantity tracking of a sample relating to the distribution of the sample between aliquots. In some examples, the report may be generated and displayed on a graphical user interface of the smartphone application discussed above.

The following paragraphs provide various examples of the embodiments disclosed herein.

Example 1 is a method including generating, with an processing device, a sample identifier for a sample, receiving, with the processing device, sample information for the sample, and receiving, with the processing device from a plurality of scientific instruments, experiment data relating to the sample and generating, with the processing device, a data structure ledger for the sample including the experiment data linked to the sample identifier and the sample information. The method also includes receiving a request for a report corresponding to the sample, querying the data structure ledger to receive ledger data corresponding to the request, and generating the report based on the ledger data received in response to the query.

Example 2 may include the subject matter of Example 1, and may further specify that the data structure ledger is a distributed ledger using a blockchain structure.

Example 3 may include the subject matter of Example 1 or 2, and may further specify that the sample information includes one or more selected from a group consisting of: a time the sample was taken, a date the sample was taken, weather at a time and location where the sample was taken, coordinates of the location where the sample was taken, a sampling technique used to retrieve the sample, and an image of the sample.

Example 4 may include the subject matter of Example 1-3, and may further specify that the experiment data includes one or more selected from a group consisting of: a weight of the sample, one or more user identifiers, one or more project identifiers, a result of an experiment, an identifier of a scientific instrument, an identifier of an experiment performed on the sample.

Example 5 may include the subject matter of Example 1-4, and may further specify that the report is generated on a smartphone application.

Example 6 may include the subject matter of Example 1-5, and may further specify that the method includes generating a barcode including the sample identifier and providing the barcode to a label printer. A label including the barcode is printed for use with the sample using the label printer.

Example 7 may include the subject matter of Example 1-6, and may further specify that the method includes receiving sample data corresponding to a change in the sample after generation of the data structure ledger and appending a timestamped sample data block to the data structure ledger in response to receiving the sample data.

Example 8 is a scientific instrument support system including one or a set of processing devices configured to generate a sample identifier for a sample, receive sample information for the sample, and generate a data structure ledger for the sample. The data structure ledger includes an initial ledger block with the sample identifier and the sample information. The one or a set of processing devices is also configured to generate an experiment ledger block in the data structure ledger. The experiment ledger block includes experiment data received from a scientific instrument. The one or a set of processing devices is further configured to receive a request for a report corresponding to the sample, query the data structure ledger to receive ledger data corresponding to the request, and generate the report based on the ledger data received in response to the query.

Example 9 may include the subject matter of Example 8, and may further specify that the data structure ledger is a distributed ledger using a blockchain structure.

Example 10 may include the subject matter of Example 8 or 9, and may further specify that the sample information includes one or more selected from a group consisting of: a time the sample was taken, a date the sample was taken, weather at a time and location where the sample was taken, coordinates of the location where the sample was taken, a sampling technique used to retrieve the sample, and an image of the sample.

Example 11 may include the subject matter of Example 8-10, and may further specify that the experiment data includes one or more selected from a group consisting of: a weight of the sample, one or more user identifiers, one or more project identifiers, a result of an experiment, an identifier of a scientific instrument, an identifier of an experiment performed on the sample.

Example 12 may include the subject matter of Example 8-11, and may further specify that the report is generated on a smartphone application.

Example 13 may include the subject matter of Example 8-12, and may further specify that the one or set of processing devices is configured to generate a barcode including the sample identifier and provide the barcode to a label printer. A label including the barcode is printed for use with the sample using the label printer.

Example 14 may include the subject matter of Example 8-13 and may further specify that the one or set of processing devices is further configured to receive a second request for a second report corresponding to the scientific instrument and query a plurality of data structure ledgers corresponding to a plurality of samples to receive second ledger data corresponding to the scientific instrument. The second ledger data includes data from a plurality of blocks of the plurality of data structure ledgers that include an identifier of the scientific instrument. The one or set of processing devices is further configured to generate the second report based on the second ledger data received in response to the query.

Example 15 is a scientific instrument support apparatus including a first logic to generate a sample identifier for a sample, second logic to receive sample information for the sample, and third logic to generate a data structure ledger for the sample. The data structure ledger includes an initial ledger block with the sample identifier and the sample information. The scientific instrument support apparatus also includes fourth logic to generate timestamped experiment ledger blocks in the data structure ledger. The experiment ledger blocks including experiment data received from a plurality of scientific instruments. The scientific instrument support apparatus further includes fifth logic to receive a request for a report corresponding to the sample, sixth logic to query the data structure ledger to receive ledger data corresponding to the request, and seventh logic to generate the report based on the ledger data received in response to the query.

Example 16 may include the subject matter of Example 15, and may further specify that the data structure ledger is a distributed ledger using a blockchain structure.

Example 17 may include the subject matter of Example 15 or 16, and may further specify that the sample information includes one or more selected from a group consisting of: a time the sample was taken, a date the sample was taken, weather at a time and location where the sample was taken, coordinates of the location where the sample was taken, a sampling technique used to retrieve the sample, and an image of the sample.

Example 18 may include the subject matter of Example 15-17, and may further specify that the experiment data includes one or more selected from a group consisting of: a weight of the sample, one or more user identifiers, one or more project identifiers, a result of an experiment, an identifier of a scientific instrument, an identifier of an experiment performed on the sample.

Example 19 may include the subject matter of Example 15-18, and may further specify that the report is generated on a smartphone application.

Example 20 may include the subject matter of Example 15-19, and may further specify that the one or set of processing devices is configured to generate a barcode including the sample identifier and provide the barcode to a label printer. A label including the barcode is printed for use with the sample using the label printer.

## Claims

1. A method for scientific instrument support, comprising:
generating, with an processing device, a sample identifier for a sample;
receiving, with the processing device, sample information for the sample;
receiving, with the processing device from a plurality of scientific instruments, experiment data relating to the sample;
generating, with the processing device, a data structure ledger for the sample including the experiment data linked to the sample identifier and the sample information;
receiving a request for a report corresponding to the sample;
querying the data structure ledger to receive ledger data corresponding to the request; and
generating the report based on the ledger data received in response to the query.

2. The method of claim 1, wherein the data structure ledger is a distributed ledger using a blockchain structure.

3. The method of claim 1 or claim 2, wherein the sample information includes one or more selected from a group consisting of: a time the sample was taken, a date the sample was taken, weather at a time and location where the sample was taken, coordinates of the location where the sample was taken, a sampling technique used to retrieve the sample, and an image of the sample.

4. The method of claim 1, claim 2 or claim 3, wherein the experiment data includes one or more selected from a group consisting of: a weight of the sample, one or more user identifiers, one or more project identifiers, a result of an experiment, an identifier of a scientific instrument, an identifier of an experiment performed on the sample.

5. The method of any preceding claim, wherein the report is generated on a smartphone application.

6. The method of any preceding claim, further comprising:
generating a barcode representative of the sample identifier;
providing the barcode to a label printer, wherein a label including the barcode is printed for use with the sample using the label printer.

7. The method of any preceding claim, further comprising:
receiving sample data corresponding to a change in the sample after generation of the data structure ledger; and
appending a timestamped sample data block to the data structure ledger in response to receiving the sample data.

8. A scientific instrument support system, comprising:
one or a set of processing devices configured to execute the method steps of any one of claims 1-7.

9. The scientific instrument support system of claim 8, wherein the one or a set of processing devices is further configured to
receive a second request for a second report corresponding to the scientific instrument; query a plurality of data structure ledgers corresponding to a plurality of samples to receive second ledger data corresponding to the scientific instrument, wherein the second ledger data includes data from a plurality of blocks of the plurality of data structure ledgers that include an identifier of the scientific instrument; and
generate the second report based on the second ledger data received in response to the query.
